Europäisches Patentamt

(19) European Patent Office

Office européen des brevets

(11) Numéro de publication : **0 280 617 B1**

(12) # FASCICULE DE BREVET EUROPEEN

(45) Date de publication du fascicule du brevet :
07.08.91 Bulletin 91/32

(51) Int. Cl.⁵ : **C07C 309/04, B01J 19/02**

(21) Numéro de dépôt : 88400362.5

(22) Date de dépôt : 17.02.88

(54) **Procédé de production d'acides alcane-sulfoniques.**

(30) Priorité : 25.02.87 FR 8702476

(43) Date de publication de la demande :
31.08.88 Bulletin 88/35

(45) Mention de la délivrance du brevet :
07.08.91 Bulletin 91/32

(84) Etats contractants désignés :
BE CH DE ES FR GB IT LI LU NL

(56) Documents cités :
R.E. KIRK et al.: "Encyclopedia of chemical
technology", vol. 13, pages 350-351, 1954, The
Interscience Encyclopedia, Inc., New York, US

(73) Titulaire : SOCIETE NATIONALE ELF
AQUITAINE (PRODUCTION)
Tour Elf, 2, Place de la Coupole, La Défense 6
F-92400 Courbevoie (FR)

(72) Inventeur : Baptiste Hubert
4 rue du domaine du chateau
F-64230 Lescar (FR)
Inventeur : Lagaude, Christian Henri
1 Rue Henri Dunant
F-64000 Pau (FR)
Inventeur : Larrouy, Michèle
1 Avenue Henri Dunant
F-64000 Pau (FR)
Inventeur : Ollivier, Jean
croix de Buzy
F-64260 Arudy (FR)

(74) Mandataire : Leboulenger, Jean et al
ATOCHEM Département Propriété Industrielle
F-92091 Paris la Défense 10 Cédex 42 (FR)

## Description

La présente invention concerne la fabrication d'acides alcane sulfoniques. Elle se rapporte plus particulièrement à la production de ces composés à partir des chlorures d'alcane sulfonyles correspondants.

Les acides alcane sulfoniques et leurs sels ont des nombreuses applications industrielles, ce qui a incité plusieurs producteurs à rechercher des procédés avantageux de fabrication de ces composés. En effet, ceux-ci trouvent des utilisations en tant que détergents, émulsifiants, catalyseurs d'estérification, en particulier pour la préparation de produits pharmaceutiques, en tant que durcisseurs de certaines résines, par exemple amino-alkydes et polyuréthanes, comme agents de finition pour métaux, etc.

Aussi connaît-on plusieurs procédés de fabrication d'acides alcane sulfoniques, par exemple oxydation d'alkyl mercaptans ou de dialkyl sulfures au moyen de l'acide nitrique ou de l'oxygène, sulfonation directe d'alcanes, traitement d'alcanes par du $SO_2$ et de l'oxygène, et l'action du chlore sur un mercaptan en présence de l'eau. Chaque procédé présente ses avantages et ses défauts ; ces derniers ont conduit certains industriels à appliquer la réaction connue de l'eau avec un chlorure d'alcane sulfonyle :

(1) ... $RSO_2Cl + H_2O \rightarrow RSO_3H + HCl$

Encyclopédie Kirk-Othmer, tome 13, pages 350-1954) qui peut se faire avec un bon rendement, commodément ; elle est économiquement intéressante lorsqu'on possède une source de $RSO_2Cl$ peu coûteux.

Un tel procédé d'hydrolyse, appliqué à du chlorure de méthane sulfonyle, est décrit dans le brevet japonais N° 48-22423 publié en 1973. Il consiste à chauffer 1 mole de $RSO_2Cl$ avec 1 à 1,5 moles $H_2O$ à une température de 100° à 140°C, pendant plusieurs heures, ce qui permet d'obtenir l'acide $RSO_3H$ avec un rendement dépassant 99,5%. Cependant, comme le produit de la réaction est coloré, le brevet japonais recommande de continuer le chauffage durant quelques heures, tout en faisant passer un courant de chlore. Celui-ci peut d'ailleurs être introduit dès le début de la réaction d'hydrolyse, ou bien seulement à la fin de cette réaction. Selon le mode opératoire appliqué, la préparation dure au total environ 2 à 7 heures, et — grâce à l'action du chlore — le produit obtenu est incolore.

Malgré les avantages qu'il présente par rapport aux autres techniques connues, ce procédé comprend cependant la sujétion qui est l'emploi d'un courant de chlore, et la nécessité de chasser ensuite l'excès de ce dernier par soufflage d'un gaz inerte. Etant donné qu'il est possible, à l'heure actuelle, de produire la matière première $RSO_2Cl$ fort économiquement, en particulier par un des procédés de la Société demanderesse (Publications françaises n° 2482591 et 2578841), le procédé d'hydrolyse décrit ci-dessus peut se montrer très avantageux si l'on arrive à s'affranchir de la sujétion du chlore susmentionnée. C'est donc dans ce sens qu'ont été orientées les recherches qui ont abouti à la présente invention.

Une première idée vers la solution du problème est venue de l'observation que le jaunissement du produit s'accentue avec le temps et devient sensible surtout vers la fin des plusieurs heures que dure l'hydrolyse ; on pourrait donc s'attendre à ne plus avoir de coloration, si l'on arrivait à effectuer l'hydrolyse très rapidement. Ainsi, la question s'est posée de savoir que faire afin d'accélérer fortement la réaction d'hydrolyse, sans une élévation nocive de température. La réponse fût obtenue à la suite d'une étude attentive de la thermodynamique de l'hydrolyse des chlorures d'alcane sulfonyles : bien que cette réaction soit exothermique (environ 53 Kcal/mol pour R = $CH_3$) le système $RSO_2Cl + H_2O$ exige une assez forte énergie d'activation (22 Kcal/mol quand R est $CH_3$) pour permettre le déclenchement de l'hydrolyse.

L'idée inventive fut donc de fournir au mélange réactionnel, dès le début, l'énergie d'activation nécessaire, sans élévation excessive de température. Cela peut s'effectuer par l'application instantanée de l'énergie libérée par le changement d'état d'un des réactifs. La forme d'exécution trouvée, la plus pratique, consiste à apporter au chlorure d'alcane sulfonyle de départ l'eau requise, sous la forme de vapeur d'eau, éventuellement surchauffée, dont la chaleur de liquéfaction produit l'activation voulue. Il suffit ensuite de laisser se poursuivre la réaction (1) une fois déclenchée, en réglant la température dans des limites adéquates, généralement entre 100° et 160°C.

Cette conception inventive, jamais proposée dans le passé, fut entièrement confirmée par l'expérience : elle a permis l'obtention d'un produit incolore, d'excellente pureté, avec rendement élevé, en moins de 45 minutes, donc 3 à 9 fois plus rapidement que ne le permet le procédé connu, rappelé plus haut. Aucune injection de chlore ou d'autre agent de décoloration n'est plus nécessaire.

Ainsi, le procédé suivant l'invention, pour la production d'un acide alcane sulfonique par hydrolyse du chlorure d'alcane sulfonyle correspondant, est-il caractérisé en ce que l'eau est apportée sous la forme de vapeur chaude, au début de la réaction, après quoi le mélange réactionnel est maintenu à la température appropriée jusqu'à ce que la majeure partie du chlorure soit transformée en l'acide alcane sulfonique, l'acide chlorhydrique formé étant éliminé au fur et à mesure de sa formation.

2

La vapeur d'eau, au moment de sa mise en contact avec RSO$_2$Cl doit être à une température égale au moins au point d'ébullition de l'eau sous la pression à laquelle on opère. Comme le procédé peut être commodément réalisé à la pression atmosphérique, la vapeur est pratiquement à 100°C, ou au-dessus, auquel cas elle est surchauffée. L'injection de la vapeur, qui se condense au contact du chlorure, a pour effet l'agitation nécessaire, en même temps que l'apport de H$_2$O pour l'hydrolyse et de l'énergie d'activation de cette réaction.

Après cette phase initiale du procédé, la réaction déclenchée se poursuivant d'elle-même, on règle la température de préférence à des valeurs entre 100° et 160°C. Dans le cas du CH$_3$SO$_2$Cl les températures les plus favorables se situent entre 125° et 145°C et surtout entre 130° et 140°C, le temps de séjour dans la zone de réaction étant généralement de 12 à 60 minutes et surtout de 15 à 45 minutes.

Un facteur important du nouveau procédé est la proportion d'eau, par rapport au chlorure de départ. Contrairement à la technique antérieure, où l'on utilise de préférence 1 à 1,5 mol. H$_2$O par mole RSO$_2$Cl, les meilleurs résultats sont obtenus avec le procédé de l'invention, lorsque ce rapport est de 1,7 à 3,5 moles H$_2$O par mole de chlorure, et de préférence 2 à 3 moles H$_2$O.

Bien que susceptible d'être réalisé en discontinu, le procédé de l'invention se prête particulièrement bien au travail en continu, fort avantageux. Il permet alors de produire, par exemple, 3,5 kg de RSO$_3$H par heure, par litre d'espace réactionnel.

Etant donné la facilité de séparation du chlorure non transformé, les RSO$_2$Cl étant en général peu solubles, ou très peu solubles dans les solutions aqueuses de RSO$_3$H obtenues, il peut être intéressant de se contenter d'une conversion du chlorure en acide de l'ordre de 80 à 90%, qui peut être obtenue extrêmement vite, par exemple en environ 20 mn. Sinon, il est possible de recycler le chlorure non transformé dans la zone de réaction. Une autre solution consiste à chauffer le chlorure restant dans une zone de réaction supplémentaire : la conversion globale peut alors approcher 100% au prix approximatif d'un doublement tout à fait acceptable de la durée totale de la production.

Le procédé de l'invention s'applique à tous les chlorures d'alcane sulfonyles pouvant être mélangés avec de l'eau à 100°C ou au-dessus ; il est fort utile à la production des acides alcane sulfoniques en C$_1$ à C$_{18}$, et en particulier en C$_1$ à C$_4$.

L'invention comprend également une installation pour la réalisation du nouveau procédé décrit. Elle concerne plus spécialement une installation pour la production en continu d'un acide alcane sulfonique.

L'installation suivant l'invention, qui comprend un réacteur pour le maintien à une température appropriée d'un mélange de l'eau avec un chlorure d'alcane sulfonyle, des moyens d'introduction continue de ces réactifs dans une entrée amont du réacteur, des moyens de sortie de l'acide sulfonique formé, et des moyens pour la séparation de l'HCl et du chlorure non converti, en aval du réacteur, est caractérisé en ce que les moyens d'introduction comprennent un mélangeur relié d'une part à une source de chlorure d'alcane sulfonyle et à une source de vapeur d'eau chaude, et — d'autre part — au réacteur lui-même.

Les moyens de séparation, en aval du réacteur, sont habituellement constitués par un condenseur pour l'HCl humide et un décanteur permettant de récupérer la fraction de chlorure initial qui n'a pas été converti.

Dans une variante, le bas du décanteur est relié à l'entrée du réacteur et est muni de moyens pour recycler, dans le réacteur, le chlorure récupéré dans le décanteur.

Selon une forme d'exécution particulière de l'invention, le décanteur est remplacé par un réacteur secondaire, dont le bas est relié à la sortie de l'acide sulfonique du réacteur principal ; ce réacteur secondaire est muni de moyens de réglage de température et il sert à parachever la réaction d'hydrolyse qui n'a pas été complète dans le réacteur principal.

A titre d'illustration non limitative, les dessins annexés représentent schématiquement trois formes d'exécution d'une installation suivant l'invention.

Fig. 1 est le schéma d'un ensemble à un seul réacteur et un séparateur, sans dispositif de recyclage du chlorure non transformé.

Fig. 2 montre un schéma semblable à celui de la figure 1, mais prévu pour le recyclage du chlorure d'alcane sulfonyle non converti.

Fig. 3 représente le cas où une installation du type de celle de la figure 1 comporte, à la place du séparateur, un réacteur secondaire pour achever l'hydrolyse du chlorure.

Sur les dessins, 1 désigne l'arrivée de chlorure d'alcane sulfonyle et 2 celle de la vapeur d'eau au point d'ébullition de l'eau, à la pression utilisée, dans un mélangeur 3. De celui-ci le mélange réactionnel, c'est-à-dire ledit chlorure et l'eau formée par la condensation de la vapeur, passe par la conduite 4 dans le réacteur principal 5.

La vapeur d'eau 2, de préférence saturante, peut être surchauffée, mais comme il est pratique de travailler sous la pression atmosphérique ou peu au-dessus, elle est en général à environ 99° à 105°C.

Le réacteur principal 5 est muni de moyens de réglage de la température, non représentés sur le dessin, qui peuvent être classiques.

3

Une solution de l'acide alcane sulfonique formé s'écoule au bas du réacteur par la tuyauterie 6, tandis que les produits volatils s'échappent en haut, par 7, pour être partiellement arrêtés dans le condenseur 8. HCl gazeux sort du condenseur par la canalisation 9, alors que d'autres constituants sont envoyés par la canalisation 10 dans un décanteur 11. Là, le restant des volatils, c'est-à-dire HCl et $H_2O$, se dégagent par 12, le chlorure non transformé, décanté, est recueilli en 13, et un peu de solution aqueuse de l'acide alcane sulfonique sort par 14. Bien entendu, le $RSO_2Cl$ résiduel peut être repris en 13 pour retourner à l'entrée 1 du mélangeur 3.

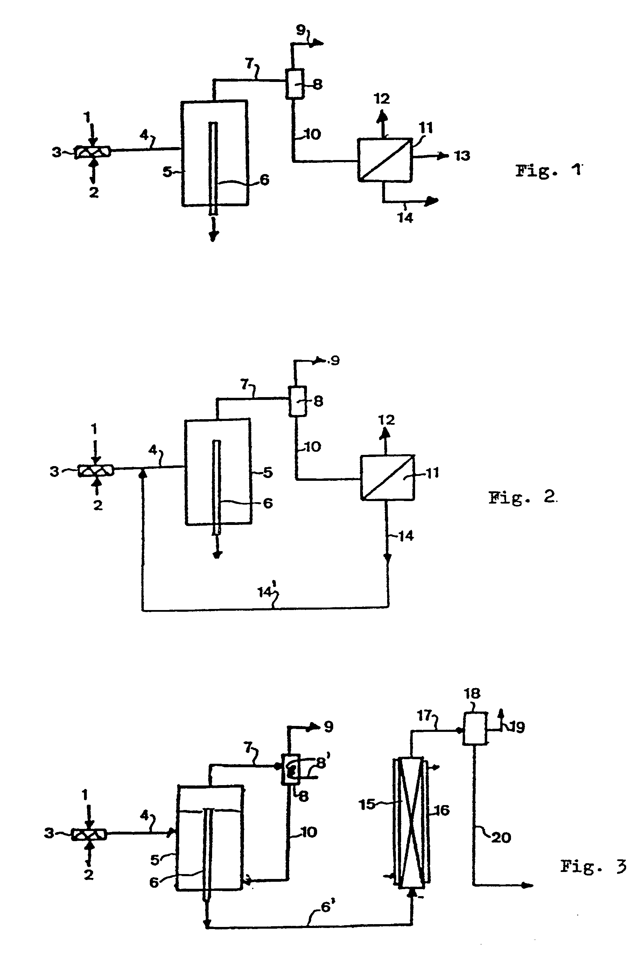

L'installation de la figure 2 diffère de celle de la figure 1 en ce que la sortie 13 de chlorure, du séparateur 11, est supprimée, tandis que la sortie 14 de ce dernier est reliée, par une conduite de recyclage 14', avec l'entrée 4 du réacteur 5. On recycle ainsi le $RSO_2Cl$ non converti, ensemble avec un peu de $RSO_3H$ aqueux qui a été entraîné à partir du réacteur par 7-8-10, jusqu'au séparateur 11.

Une troisième forme d'exécution de l'invention est représentée sur la figure 3. Ici, la solution de l'acide sulfonique, s'écoulant par 6 du réacteur principal 5, est envoyée par une canalisation 6' au bas d'un réacteur secondaire 15 où s'achève l'hydrolyse de $RSO_2Cl$ non transformé dans le réacteur principal 5. Des moyens 16 sont prévus pour régler convenablement la température dans le réacteur secondaire 15, le plus souvent à une valeur du même ordre que dans 5, ou quelque peu supérieure.

Comme la présence de HCl est défavorable au déroulement de l'hydrolyse (1), il est important de l'éliminer autant que possible du mélange envoyé dans le réacteur secondaire 15 ; c'est pourquoi l'installation comprend — de même que celles des figures 1 et 2 — le circuit de dégazage de l'HCl, 7-8-9. Toutefois, le réfrigérant 8', employé dans le condenseur 8, est prévu pour arrêter tout le $RSO_2Cl$ entraîné et l'eau ; ces matières retournent au bas du réacteur principal 5 par la canalisation 10'.

Un autre séparateur de l'HCl 18 surmonte le réacteur secondaire 15 et communique avec lui par une liaison 17. HCl sort en 19, tandis que l'acide sulfonique produit, contenant de l'eau et peu de HCl, s'écoule par 20.

Les exemples non limitatifs, qui suivent, montrent la préparation en continu de $CH_3SO_3H$ par hydrolyse de $CH_3SO_2Cl$, selon chacune des trois variantes décrites plus haut.

Dans ces exemples, le mélangeur 3 est un tube de 6 mm de diamètre et il a une capacité de 10 ml. Le réacteur principal 5 est constitué par une colonne de 60 mm de diamètre, haute de 250 mm, garnie d'anneaux de telle sorte que son volume utile est de 200 ml.

Le condenseur 8 est à 27°C.

EXEMPLE 1

L'installation est celle de la figure 1 employée en continu, dans les conditions suivantes :

```
débit de CH₃SO₂Cl (en 1) ....... 847,3 g/h=7,4 moles/h
   "   de vapeur à 102°C (en 2).. 360   g/h=20  moles/h
   soit 2,7 moles H₂O par mole de chlorure
température (en 5)  .............150°C.
```

Résultats obtenus :

```
production de CH₃SO₃H (en 6) .. 585   g/h=6,09moles/h,
         "        soit 2,93 kg par litre d'espace utile du
                  réacteur/h,
         "        rendement 82,3% sur le chlorure,
récupération du HCl (en 9) .... 60%
```

Temps de séjour du mélange réactionnel :

```
dans le mélangeur (3) ......... 45 secondes
   "   le réacteur  (5) ......... 16 mn 45 sec.
```

4

Ainsi plus de 82% de chlorure de départ ont été convertis en 17 mn 30 s, alors qu'il eut fallu pour cela plusieurs heures dans la technique antérieure.

EXEMPLE 2

La préparation en continu est effectuée dans le dispositif de la figure 2, c'est-à-dire avec recyclage du chlorure de méthane sulfonyle qui n'a pas réagi.

```
Débit de CH3SO2Cl frais(en1)..  834,7 g/h = 7,29 mol/h
     "        " recyclé(en 14'-4).  125,9  "  = 1,10   "
     " total   "                    960,6 g/h = 8,39 mol/h
Débit de vapeur H2O (en 2)....  260   g/h =14,45 mol/h
     à 102°C
     soit 1,72 mol.H2O par mole de chlorure.


          Température (en 5) ...... 136°C.
```

Résultats :

```
    production de CH3SO3H (en 6) ... 702 g/h = 7,32 mol/h
     soit 3,5 kg par h par litre d'espace utile ;
    rendement  sur le chlorure utilisé (en 4) ... 87,1%
    récupération de HCl (en 9) ................... 75 %.
```

Temps de séjour du mélange réactionnel dans :

```
    le mélangeur (3) ............ 45 secondes
    le réacteur  (5) .............14 mn 30 sec.
                     total  ...... 15 mn 15 sec.
```

Le recyclage du chlorure permet donc d'augmenter le rendement en acide sulfonique (87,1% contre 82,3% dans l'exemple 1) et d'atteindre ce résultat en 15 mn 15 sec.

Il est à noter que, déjà pendant les premières 45 secondes dans le mélangeur (3), 25% environ du chlorure introduit sont convertis en acide sulfonique.

EXEMPLES 3 à 5

On opère avec recyclage du chlorure non transformé, dans l'appareillage de la figure 2, comme à l'exemple 2, mais à plus basse température, 110°-114°C, en faisant varier la proportion de H2O par rapport au CH3SO2Cl.

| Exemple n° | 3 | 4 | 5 |
|---|---|---|---|
| Rapport molaire $H_2O/CH_3SO_2Cl$ .... | 2,84 | 1,73 | 1,5 |
| Rendement en $CH_3SO_3H$ .. % ... | 80 | 72,2 | 52,7 |

Il en résulte l'intérêt d'avoir plus de 2 moles H2O par mole de chlorure utilisé.

D'autre part, la comparaison de l'exemple 4 avec l'exemple 2 montre l'important effet de la température :

5

avec la même proportion d'eau de 1,73 on a un rendement de 87,1% à 136°C contre 72,2% à 110-114°C.

## EXEMPLES 6 à 8

Les essais sont encore conduits comme dans l'exemple 2, avec le dispositif de la figure 2, comportant le recyclage du chlorure non consommé. On opère avec une durée de séjour un peu plus longue dans le réacteur 5, à savoir 17 mn 30 sec. (plus 45 sec. dans le mélangeur), soit une alimentation en $CH_3SO_2Cl$ de 767 g/h (= 6,7 mol/h). La proportion de $H_2O$ est de 2 moles par mole de chlorure.

Résultats :

| Exemple n°........... | 6 | 7 | 8 |
|---|---|---|---|
| Température ........ | 122°-130° | 137°-138° | 143°-148° |
| Rendement % ........ | 93,7 | 94,3 | 93,2 |

Dans les conditions susindiquées, l'optimum de température se situe donc vers 137°C.

## EXEMPLES 9 et 10

La préparation d'acide méthane sulfonique en continu est réalisée dans le dispositif de la figure 3, c'est-à-dire avec un réacteur (15) secondaire, décrit plus haut, en plus du réacteur principal (5). Ce réacteur supplémentaire a une capacité de 245 ml (hauteur 40 cm). Les conditions opératoires et les résultats sont résumés ci-après.

| Exemple n° | 9 | 10 |
|---|---|---|
| Débit de $CH_3SO_2Cl$ g/h à 25°C ........... | 680 | 744 |
| " de vapeur d'eau g/h à 100°C/1 bar | 220 | 252 |
| Rapport molaire eau/chlorure .......... | 2,05 | 2,15 |
| Temps de séjour dans le : | | |
| mélangeur (3) .................... | 0'45" | 0'45" |
| réacteur (5) .................... | 19'15" | 17'32" |
| réacteur secondaire (15) .......... | 22'00 | 22'00 |
| total .................... | 42' − | 40'17" |
| Température dans le réacteur (5) ..... | 130°C | 131°C |
| " " réacteur secondaire (15) | 130°C | 133°C |
| Production en kg par h, par l d'espace | | |
| total de réaction .................. | 1,2 | 1,3 |
| Rendement % ..................... | 100% | 99,5% |

On voit que l'hydrolyse peut être pratiquement complète, si l'on utilise un réacteur supplémentaire, après la séparation de l'HCl.

## EXEMPLE 11 (Comparatif)

Les opérations de l'exemple 10 sont répétées, mais à la place de la vapeur d'eau saturante sous 1 bar, à 100°C, on introduit dans le mélangeur (3) de l'eau liquide à 100°C.

Le rendement en acide méthane sulfonique est alors de 24%. Pour arriver à un rendement de 99% il a fallu abaisser le débit de $CH_3SO_2Cl$ de 744 à 180 g/h, c'est-à-dire laisser le mélange à 131°-133°C pendant 166 minutes (au lieu de 40'17" dans l'exemple 10) et alors le produit obtenu était jaune. Cela illustre l'effet remarquable de l'emploi de la vapeur d'eau.

EXEMPLES 12 à 14

Dans le même dispositif et suivant les mêmes conditions opératoires qu'à l'exemple 9, on a hydrolysé les sulfochlorures d'éthane, de propane-1 et de butane-1 au débit de 680 g/h. Les résultats obtenus sont rassemblés dans le tableau suivant :

| EXEMPLES | 12 | 13 | 14 |
|---|---|---|---|
| Sulfochlorure de............. | éthane | propane-1 | butane-1 |
| Acide produit (g/h).......... | 582 | 592 | 600 |
| Production (kg/h/l).......... | 1,23 | 1,25 | 1,26 |
| Rendement (%)................ | 100 | 100 | 100 |

**Revendications**

1. Procédé de fabrication d'un acide alcane sulfonique par l'hydrolyse à chaud d'un chlorure d'alcane sulfonyle, de préférence continu, caractérisé en ce que l'eau est introduite au début de la réaction, sous la forme de vapeur d'eau dont la température est au moins égale au point d'ébullition de l'eau sous la pression du système réactionnel, et que l'acide chlorhydrique formé est éliminé au fur et à mesure de sa formation.

2. Procédé selon la revendication 1, caractérisé en ce que la proportion de vapeur d'eau utilisée est de 1,7 à 3,5 moles $H_2O$ par mole de chlorure d'alcane sulfonyle, et plus particulièrement 2 à 3 moles.

3. Procédé selon la revendication 1 ou 2, caractérisé en ce que la vapeur d'eau est introduite à une température voisine de 100°C, en particulier entre 99° et 105°C, après quoi le mélange réactionnel est maintenu à une température comprise entre 100° et 160°C et de préférence entre 125° et 145°C.

4. Procédé selon l'une des revendications 1 à 3, dans lequel l'acide chlorhydrique est séparé du produit de la réaction, à l'état de phase gazeuse dont on sépare, par condensation, le chlorure non transformé, caractérisé en ce que ce dernier est recyclé dans la zone de réaction.

5. Procédé suivant la revendication 4, caractérisé en ce qu'après la séparation de HCl, le produit de réaction est passé dans une seconde zone de réaction pour achever l'hydrolyse.

6. Procédé selon l'une des revendications 1 à 5, caractérisé en ce que le temps de séjour du mélange dans la ou les zones de réaction est de 12 à 60 minutes et, en particulier, de 15 à 45 minutes.

7. Installation pour la réalisation du procédé selon l'une des revendications 1 à 6, qui comprend un réacteur (5), des moyens d'introduction continue (1, 2) de réactifs dans l'entrée amont (4) du réacteur (5), des moyens de sortie de l'acide alcane sulfonique (6) et des moyens de séparation de HCl et du chlorure non converti (7, 8, 9, 10, 11) en aval du réacteur (5), caractérisé en ce qu'un mélangeur (3) est intercalé entre les introductions (1, 2) et l'entrée amont (4), et que l'entrée prévue pour l'eau (2) est reliée à une source de vapeur d'eau chaude.

8. Installation suivant la revendication 7, caractérisée en ce que les moyens de séparation du chlorure non converti (11), en particulier un décanteur, sont reliés (14-14') au réacteur (5).

9. Installation suivant la revendication 8, caractérisée en ce que la sortie (6) du produit du réacteur (5) est reliée (6') au bas d'un réacteur secondaire (15), dont la sortie (17) communique avec un séparateur de HCl (18).

**Claims**

1. Process for the production of an alkanesulphonic acid by the, preferably continuous, hot hydrolysis of an alkanesulphonyl chloride, characterized in that the water is introduced at the start of the reaction in the form of water vapour, the temperature of which is at least equal to the boiling point of water under the pressure of the reaction system, and in that the hydrochloric acid formed is removed at the rate at which it is formed.

2. Process according to Claim 1, characterized in that the proportion of water vapour used is from 1.7 to 3.5 moles of $H_2O$ per mole of alkanesulphonyl chloride and more particularly 2 to 3 moles.

3. Process according to Claim 1 or 2, characterized in that the water vapour is introduced at a temperature

7

close to 100°C, in particular between 99° and 105°C, after which the reaction mixture is maintained at a temperature of between 100° and 160°C and preferably between 125° and 145°C.

4. Process according to one of Claims 1 to 3, in which the hydrochloric acid is separated from the reaction product, in the gas phase state, from which the unconverted chloride is separated by condensation, characterized in that the said unconverted chloride is recycled into the reaction zone.

5. Process according to Claim 4, characterized in that after the separation of HCl, the reaction product is passed into a second reaction zone in order to achieve the hydrolysis.

6. Process according to one of Claims 1 to 5, characterized in that the residence time of the mixture in the reaction zone or zones is from 12 to 60 minutes and in particular from 15 to 45 minutes.

7. Installation for carrying out the process according to one of Claims 1 to 6, which comprises a reactor (5), means for continuous introduction (1, 2) of reactants into the upstream inlet (4) of the reactor (5), outlet means for the alkanesulphonic acid (6) and means for separating HCl and the unconverted chloride (7, 8, 9, 10, 11) downstream of the reactor (5), characterized in that a mixer (3) is interposed between the introductions (1, 2) and the upstream inlet (4) and in that the inlet provided for the water (2) is connected to a source of hot water vapour.

8. Installation according to Claim 7, characterized in that the means for separating the unconverted chloride (11), in particular a settler, are connected (14-14') to the reactor (5).

9. Installation according to Claim 8, characterized in that the outlet (6) of the product from the reactor (5) is connected (6') to the bottom of a secondary reactor (15), the outlet (17) of which communicates with an HCl separator (18).

## Patentansprüche

1. Verfahren zur vorzugsweise kontinuierlichen Herstellung einer Alkansulfonsäure durch heiße Hydrolyse eines Alkansulfonylchlorids, dadurch gekennzeichnet, daß das Wasser zu Reaktionsbeginn in Form von Wasserdampf, dessen Temperatur wenigstens gleich dem Siedepunkt des Wassers unter Reaktionsdruck ist, eingebracht und daß die gebildete Chlorwasserstoffsäure in dem Maße ihrer Bildung entfernt wird.

2. Verfahren nach Anspruch 1, dadurch gekennzeichnet, daß der Anteil des verwendeten Wasserdampfs 1,7 bis 3,5 mol und insbesondere 2 bis 3 mol H$_2$O pro mol Alkansulfonylchlorid beträgt.

3. Verfahren nach Anspruch 1 oder 2, dadurch gekennzeichnet, daß der Wasserdampf mit einer Temperatur nahe 100°C und insbesondere zwischen 99°C und 105°C eingebracht und danach das Reaktionsgemisch bei einer Temperatur zwischen 100°C und 160°C und vorzugsweise zwischen 125°C und 145°C gehalten wird.

4. Verfahren nach einem der Ansprüche 1 bis 3, in dem die Chlorwasserstoffsäure vom Reaktionsprodukt als Gasphase abgetrennt wird, aus der man durch Kondensation das nicht umgewandelte Chlorid abscheidet, dadurch gekennzeichnet, daß letzteres in die Reaktionszone recycliert wird.

5. Verfahren nach Anspruch 4, dadurch gekennzeichnet, daß nach der HCl-Abtrennung das Reaktionsprodukt in eine zweite Reaktionszone zur Vervollständigung der Hydrolyse geleitet wird.

6. Verfahren nach einem der Ansprüche 1 bis 5, dadurch gekennzeichnet, daß die Verweilzeit des Gemischs in der oder den Reaktionszone(n) 12 bis 60 min und insbesondere 15 bis 45 min beträgt.

7. Anlage zur Durchführung des Verfahrens nach einem der Ansprüche 1 bis 6, umfassend einen Reaktor (5), Einrichtungen zur kontinuierlichen Zuführung (1, 2) der Ausgangsstoffe in den oberen Eingang (4) des Reaktors (5), Einrichtungen zum Abführen der Alkansulfonsäure (6) und Einrichtungen zur Trennung der HCl und des nicht umgewandelten Chlorids (7, 8, 9, 10, 11) am unteren Ende des Reaktors (5), dadurch gekennzeichnet, daß ein Mischer (3) zwischen die Zuführungseinrichtungen (1, 2) und den oberen Eingang (4) geschaltet ist und daß der für Wasser vorgesehene Eingang (2) mit einer Versorgung für heißen Wasserdampf verbunden ist.

8. Anlage nach Anspruch 7, dadurch gekennzeichnet, daß die Einrichtungen zur Abtrennung des nicht umgewandelten Chlorids (11), insbesondere ein Abscheider, durch (14-14') mit dem Reaktor (5) verbunden sind.

9. Anlage nach Anspruch 8, dadurch gekennzeichnet, daß der Ausgang (6) des Produkts des Reaktors (5) durch (6') mit dem Boden eines zweiten Reaktors (15) verbunden ist, dessen Ausgang (17) zum HCl-Abscheider (18) führt.

Fig. 1

Fig. 2

Fig. 3